# EUROPÄISCHE PATENTSCHRIFT

(11) **EP 1 212 032 B1**
(45) Veröffentlichungstag und Bekanntmachung des Hinweises auf die Patenterteilung: **04.05.2005**
(21) Anmeldenummer: 00971277.9
(22) Anmeldetag: 08.09.2000
(51) Int. Cl.: A61K 7/13, C07C 211/29

(54) **NEUE DIREKTZIEHENDE HAARFARBSTOFFE**
NEW DIRECT HAIR COLORANTS
NOUVEAUX COLORANTS DIRECTS POUR LES CHEVEUX

(30) Priorität: 17.09.1999 DE 19944528
(43) Veröffentlichungstag der Anmeldung: 12.06.2002
(73) Patentinhaber: Henkel Kommanditgesellschaft auf Aktien, 40589 Düsseldorf (DE)
(72) Erfinder: NAUMANN, Frank, 40593 Düsseldorf (DE); ROSE, David, 40723 Hilden (DE); MEINIGKE, Bernd, 51381 Leverkusen (DE); HÖFFKES, Horst, 40595 Düsseldorf (DE)
(86) Internationale Anmeldenummer: PCT/EP2000/008774
(87) Internationale Veröffentlichungsnummer: WO 2001/021144

(56) Entgegenhaltungen:
- DE-A- 3 528 882
- US-A- 3 632 582
- US-A- 3 944 612
- US-A- 5 414 128

## Beschreibung

Die Erfindung betrifft neue direktziehende Farbstoffe, Mittel zum Färben und Tönen keratinischer Fasern, insbesondere menschlicher Haare, mit diesen Farbstoffen sowie die Verwendung dieser Mittel.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Gute Farbstoffe zeichnen sich durch hohe Farbstärke aus. Weiterhin sind gute Schweiß-, Wasch- und Lichtechtheit gewünscht. Ferner sollten sie in toxikologischer und dermatologischer Einsicht unbedenklich sein. Es ist auch von Vorteil, wenn die Substanzen eine hohe Löslichkeit in verschiedenen Basisformulierungen besitzen.

Färbemittel auf Basis von Oxidationsfarbstoffen führen zu brillanten und dauerhaften Farbtönen. Sie bedingen allerdings den Einsatz starker Oxidationsmittel, wie beispielsweise Wasserstoffperoxid-Lösungen. Dies kann das zu färbende Haar schädigen. Diesen Schädigungen muß dann mit entsprechenden Pflegeprodukten entgegengewirkt werden. Außerdem können Kontakte der Haut mit diesen Färbemitteln bei sehr empfindlichen Personen zu unerwünschten Reaktionen führen.

Färbemittel auf der Basis direktziehender Farbstoffe kommen ohne Oxidationsmittel aus und können bei pH-Werten im Bereich des Neutralpunktes formuliert werden. Ein wesentlicher Nachteil der Färbemittel auf Basis direktziehender Farbstoffe ist die geringe Waschechtheit der erzielten Färbungen. Das Aufziehvermögen der Farbstoffmoleküle auf das Haar sowie der Glanz der gefärbten Haare, können in vielen Fällen ebenfalls nicht voll befriedigen.

Zur Entwicklung modischer Tönungen ist es notwendig rote Farbtöne zu erzielen. Dies wird häufig durch den Einsatz von 2-Nitro-p-phenylendiaminderivaten erreicht. Diese Derivate sind jedoch häufig in Wasser nur unzureichend löslich oder dispergierbar. Wenn der Farbstoff im Färbemedium nicht solubilisierbar ist, führt dies zu ungleichmäßigen Färbungen und es besteht ein hohes Risiko, nur schwache Ausfärbungen zu erreichen.

Zur Verbesserung der Egalisierung sowie zur Erweiterung des Nuancenspektrums werden direktziehende Farbstoffe häufig auch in Oxidationshaarfarben eingesetzt. Allerdings weisen die üblicherweise eingesetzten direktziehenden Farbstoffe nur eine geringe Stabilität gegen Reduktions- und Oxidationsmittel auf.

Es besteht daher weiterhin Bedarf an direktziehenden Farbstoffen, die die oben beschriebenen Nachteile nicht aufweisen.

Es wurde nun überraschenderweise gefunden, daß bestimmte 2-Nitro-p-phenylendiamin-Derivate die an direktziehende Farbstoffe gestellten Anforderungen in einem hohen Maße erfüllen.

Ein erster Gegenstand der vorliegenden Erfindung sind daher Mittel zum Färben keratinischer Fasern, insbesondere menschlicher Haare, die in einem zum Färben geeigneten Träger mindestens ein 2-Nitro-p-phenylendiaminderivat der Formel (I) 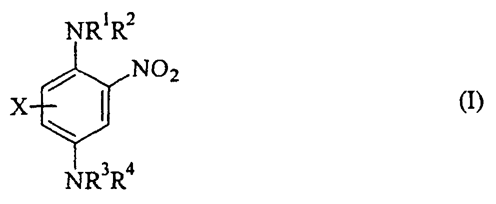 in der R¹ bis R⁴ voneinander unabhängig stehen für Wasserstoff, eine C₁₋₄-Hydroxyalkylgruppe oder einen gesättigten, einfach oder mehrfach ungesättigten C₇₋₈-Ring, der gegebenenfalls mit einer C₁₋₄-Alkylgruppe, einem Halogenatom, einer Hydroxygruppe oder einer Aminogruppe substituiert sein kann, und X steht für Wasserstoff oder ein Halogenatom, mit der Maßgabe, daß mindestens einer der Substituenten R¹ bis R⁴ ein C₇₋₈-Ring ist, enthalten.

Beispiele für die als Substituenten in den erfindungsgemäßen Verbindungen genannten, C₁- bis C₄-Alkylgruppen sind die Gruppen Methyl, Ethyl, Propyl, Isopropyl und Butyl. Ethyl und Methyl sind bevorzugte Alkylgruppen. Eine Methylgruppe ist besonders bevorzugt. Als bevorzugte Beispiele für eine C₁- bis C₄-Hydroxyalkylgruppe können eine Hydroxymethyl-, eine 2-Hydroxyethyl-, eine 3-Hydroxypropyl- oder eine 4-Hydroxybutylgruppe genannt werden, eine 2-Hydroxyethylgruppe ist besonders bevorzugt. Beispiele für ein Halogenatom sind erfindungsgemäß ein F-, ein Cl- oder ein Br-Atom, ein Cl-Atom ist besonders bevorzugt. Bevorzugte Ringsysteme sind der Cycloheptylring, der Cycloocytlring sowie deren methyl-, hydroxy-, oder amino-substituierten Derivate. Der unsubstituierte Cycloheptylring und der unsubstituierte Cyclooctylring sind besonders bevorzugt.

Besonders bevorzugt sind Verbindungen der Formel (I), bei denen die Reste R² bis R⁴ für Wasserstoff stehen. Dennoch sind auch Verbindungen der Formel (I), bei denen R¹ und R³ jeweils für einen Ringsubstituenten stehen, und Verbindungen, bei denen einer der Reste R¹ oder R³ für ein Ringsystem und der andere der Reste R¹ oder R³ für eine 2-Hydroxyethylgruppe stehen, bevorzugt.

Bevorzugt sind ferner Verbindungen der Formel (I), die einen Chlorsubstituenten (X) an der Position 3, 5 oder 6 tragen.
Besonders bevorzugt sind Verbindungen der Formel (I), bei denen X für ein Wasserstoffatom steht.

Ganz besonders bevorzugte Verbindungen der Formel (I) sind 1-(N-cycloheptylamino)-2-nitro-4-aminobenzol und 1-(N-cyclooctylamino)-2-nitro-4-aminobenzol.

Gemäß einer ersten bevorzugten Ausführungsform der Erfindung handelt es sich um solche Mittel, die nur eine vorübergehende Färbung der Faser bewirken sollen. Solche Mittel werden häufig als Tönungsmittel bezeichnet. Diese Ausfihrungsform umfaßt beispielsweise auch solche Haarbehandlungsmittel, mit denen die Haare nicht nur vorübergehend gefärbt, sondern auch zu einer bestimmten Frisur gestylt werden sollen. In diesem Falle spricht man von Tönungsfestigem.

Da solche Mittel üblicherweise ohne die Zuhilfenahme von oxidierenden Komponenten, insbesondere Wasserstoffperoxid, formuliert werden, können die erfindungsgemäßen Mittel gemäß dieser Ausführungsform frei von Oxidationsfarbstoffvorprodukten sein.

Wenngleich die Verbindungen gemäß Formel (I) auch als alleinige Farbstoffkomponente eingesetzt werden können, so enthalten die Mittel gemäß dieser Ausführungsform bevorzugt noch mindestens einen weiteren Farbstoff vom Typ der Direktzieher.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5.6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Gemäß einer zweiten bevorzugten Ausführungsform handelt es sich bei den beanspruchten Mitteln um Haarfärbemittel für die dauerhafte Färbung der Haare. Diese Mittel enthalten mindestens ein Oxidationsfarbstoffvorprodukt vom Typ der Entwickler. Diese, in der Regel farblosen, Verbindungen reagieren unter der Einwirkung von Oxidationsmitteln oder von Luftsauerstoff, gegebenenfalls mit Hilfe spezieller Enzyme oder Metallionen als Katalysator, mit sich unter der Ausbildung der gewünschten Farbstoffe. Insbesondere zur Ausbildung natürlicher Haarfarbtöne werden aber in der Regel Kombinationen von mehreren Entwicklerkomponenten eingesetzt. Weiterhin werden in der Regel zusätzlich sogenannte Kuppler eingesetzt, die unter dem Einfluß von Oxidationsmitteln mit den Entwicklerkomponenten reagieren, was zu neuen Farben bzw. einer Nuancierung der Farbe führt. Erfindungsgemäß kann sowohl eine Kupplerkomponente als auch mehrere Kupplerkomponenten in Kombination mit einer oder mehreren Entwicklerkomponenten eingesetzt werden.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol.

Besonders bevorzugte Entwicklerkomponenten sind erfindungsgemäß p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß bevorzugte Kupplerkomponenten sind ferner 3-Dimethylaminophenol, 2,4-Dihydroxyanilin, 8-Amino-6-methoxy-chinolin, 2-Amino-5-naphthol-1,7-disulfonsäure, 3,5-Diaminobenzamid, 3-Methylsulfonylamino-2-methylanilin, 5,6-Dihydroxybenzimidazol, 2,2'-Dihydroxybenzylamin, 3,5,3',5'-Tetraamino-2,2'-dimethoxy-diphenyl, 3,5-Diamino-p-chlorbenzotrifluorid, 4-Methyl-3-aminophenol, 2,4-Diamino-3-chlorphenol, 1-Amino-3-di-(2-hydroxyethylamino)-4-ethoxybenzol, 2,4-Dimethylresorcin, Bis-(2,4-diaminophenoxy)-methan, 2,6-Bis-(hydroxyethyl)-pyridin, 4-Hydroxy-3-methoxybenzylalkohol, 8-Hydroxychinolin, 4-Hydroxy-3-methoxy-benzylamin, 4-Ethylresorcin, 2-Methylthio-5-aminophenol, 5-[(3-Hydroxypropyl)amino]-2-methylphenol, 2,6-Dimethoxy-3-aminophenol und 2,6-Diamino-3-methylthiotoluol.
Erfindungsgemäß besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Erfindungsgemäß können solche Entwickler- und Kupplerkomponenten, die für die Ausbildung der Färbung keine Oxidationsmittel außer Luftsauerstoff benötigen, bevorzugt sein.

Die erfindungsgemäßen Mittel dieser Ausführungsform können außer den Verbindungen gemäß Formel (I), den Entwickler und gegebenenfalls Kupplerkomponenten gewünschtenfalls zur Nuancierung noch weitere direktziehende Farbstoffe enthalten. Es sei an dieser Stelle auf die oben angegebene Auflistung verwiesen.

Die erfindungsgemäßen Haarfärbemittel enthalten sowohl die Entwicklerkomponenten als auch die Kupplerkomponenten, bevorzugt in einer Menge von 0,005 bis 20 Gew.-%, jeweils bezogen auf das Färbemittel ohne die Oxidationsmittelzubereitung. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer bevorzugten Ausführungsform enthalten die erfindungsgemäßen Mittel, insbesondere, wenn sie noch konditionierende oder festigende Eigenschaften aufweisen sollten, weiterhin anionische, nichtionogene oder insbesondere kationische Polymere.

Als konditionierende Wirkstoffe geeignete kationische Polymere enthalten innerhalb des Polymergerüstes kationische Gruppen. Diese Gruppen können Teil der Polymerkette sein, sie können sich aber auch in Seitenketten befinden, die über Zwischenglieder mit einer Hauptkette verbunden sind. Übliche kationische Gruppen enthalten quartäre Stickstoffoder Phosphoratome. Gruppen mit quartären Stickstoffatomen sind dabei bevorzugt. Die quartären Stickstoffatome können dabei sowohl 4 unterschiedliche oder z. T. gleiche Substituenten tragen, als auch Teil eines Ringsystems sein. Bevorzugte kationische Gruppen sind Ammonium- und Imidazoliumgruppen.

Beispiele für solche Polymere sind:
- quaternierte Cellulose-Derivate, wie sie unter den Bezeichnungen Celquat® und Polymer JR® im Handel erhältlich sind. Die Verbindungen Celquat® H 100, Celquat L 200 und Polymer JR®400 sind bevorzugte quaternierte Cellulose-Derivate.
- Copolymere des Vinylpyrrolidons mit quaternierten Derivaten des Dialkylaminoalkylacrylats- und -methacrylats, wie beispielsweise mit Diethylsulfat quaternierte Vinylpyrrolidon-Dimethylaminoalkylmethacrylat-Copolymere sowie das Vinylpyrrolidon-Methacrylamidopropyltrimethylammoniumchlorid-Copolymere. Solche Verbindungen sind unter den Bezeichnungen Gafquat®734, Gafquat®755 bzw. Gafquat® HS 100 im Handel erhältlich.
- Copolymerisate des Vinylpyrrolidons mit Vinylimidazoliummethochlorid, wie sie unter der Bezeichnung Luviquat® angeboten werden.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- quaternierte Guar-Derivate, wie sie unter den Bezeichnungen Cosmedia Guar® und Jaguar® im Handel erhältlich sind. Bevorzugte Guar-Derivate sind beispielsweise Cosmedia Guar® C-261 und Jaguar® C 13-S.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).
- Chitosan und dessen Derivate
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquatemium-2, Polyquaternium-10 und Polyquaternium-22.

Bevorzugt sind die kationischen Polymere in den erfindungsgemäßen Mitteln in Mengen von 0,1 - 5 Gew.-%, bezogen auf die gesamte Zubereitung, enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.

Beispiele für geeignete anionische Polymere sind:
- Copolymere der Acrylsäure und/oder Methacrylsäure oder deren Ester mit C₁₀₋₃₀-Alkylacrylaten, wie sie beispielsweise unter der Bezeichnung Pemulen® vertrieben werden.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind unter den Markenbezeichnungen Resyn® (NATIONAL STARCH), Luviset® (BASF) und Gafset® (GAF) im Handel; die Produkte Luviset®CA-66 und Luviset®CAP können bevorzugt sein.
- Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold® strong (BASF) vertrieben werden, sowie Methacrylsäure/Ethylacrylat/t-Butylacrylat-Terpolymere, die unter der Bezeichnung Luvimer® 100P (BASF) vertrieben werden.

Die Verwendung von anionischen, nichtionogenen oder kationischen Polymeren kann in solchen Haarfärbemitteln bevorzugt sein, die frei von Oxidationsfarbstoffvorprodukten sind.

Zur Herstellung der erfindungsgemäßen Färbemittel werden die direktziehenden Farbstoffe und gegebenenfalls die Oxidationsfarbstoffvorprodukte in einen geeigneten wasserhaltigen Träger eingearbeitet. Zum Zwecke der Haarfärbung sind solche Träger z. B. Cremes, Emulsionen, Gele oder auch tensidhaltige schäumende Lösungen, z. B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.
Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. In diesem Zusammenhang sei auf die oben genannten Polymere verwiesen.

Als konditionierende Wirkstoffe weiterhin geeignet sind Silikonöle, insbesondere Dialkylund Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in, für diesen Zweck üblichen Mengen, eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Es ist prinzipiell möglich, die erfindungsgemäßen Mittel so zu formulieren, daß sie entweder auf dem Haar verbleiben oder wieder aus dem Haar ausgespült werden.

Gemäß einer bevorzugten Ausführungsform werden die erfindungsgemäßen Mittel so formuliert, daß sie auf dem Haar verbleiben können. Dies ist insbesondere bei sogenannten Tönungsmitteln der Fall, aber auch bei Mitteln, die außerdem eine festigende Funktion ausüben sollen.

Ein zweiter Gegenstand der Anmeldung ist die Verwendung der erfindungsgemäßen Mittel zum Färben und Tönen von keratinischen Fasern, insbesondere menschlichen Haaren.
Ein dritter Gegenstand der vorliegenden Erfindung sind die Verbindungen 1-(Ncycloheptylamino)-2-nitro-4-aminobenzol und 1-(N-cyclooctylamino)-2-nitro-4-aminobenzol.

Die nachfolgenden Beispiele sollen den Erfmdungsgegenstand näher erläutern.

### Beispiele

### 1. Herstellung von 1-(N-cycloheptylamino)-2-nitro-4-aminobenzol

Eine Mischung aus 3,12g (0,020mol) 4-Fluor-3-nitroanilin, 2,28g (0.022mol) Cycloheptylamin und 1,7g (0,020mol) Natriumhydrogencarbonat wurden in 5ml Wasser und 5ml 2-Propanol für 3h refluxiert. Nach Abkühlen auf Raumtemperatur wurden die Rückstände abfiltriert und das Filtrat mit Chloroform extrahiert. Das Lösemittel wurde im Vakuum entfernt und der Rückstand aus 20%iger Salzsäure umkristallisiert. Es wurde ein grünbrauner Feststoff erhalten, der bei 153°C schmilzt.

### 2. Herstellung von 1-(N-cyclooctylamino)-2-nitro-4-aminobenzol

Eine Mischung aus 3,12g (0,020mol) 4-Fluor-3-nitroanilin, 2,57g (0,022mol) Cyclooctylamin und 1,7g (0,020mol) Natriumhydrogencarbonat wurden in 5ml Wasser und 5ml 2-Propanol für 3h refluxiert. Nach Abkühlen auf Raumtemperatur wurden die Rückstände abfiltriert und das Filtrat mit Chloroform extrahiert. Das Lösemittel wurde im Vakuum entfernt und der Rückstand aus 20%iger Salzsäure umkristallisiert. Es wurde ein gelbgrüner Feststoff erhalten, der bei 161°C schmilzt.

### 3. Haarfärbecremes mit direktziehendem Farbstoff

| *Teilmischung A* | |
|---|---|
| Cetearylalkohol | 1,00g |
| Fettalkoholgemisch auf Basis von Kokosnußöl | 1,00g |
| Akypo® RLM 45N¹ | 1,10g |
| p-Hydroxybenzoesäurepropylester | 0,05g |
| p-Hydroxybenzoesäuremethylester | 0,15g |
| Wasser | 70,00g |

| | |
|---|---|
| ¹ Laurylalkohol mit ca. 4,5mol Ethylenoxid-Essigsäure-Natriumsalz (ca.82% Aktivsubstanz; INCI-Bezeichnung: Sodium Laureth-6 Carboxylate) (KAO, Chem-Y) | |

Die Substanzen wurden bei 80°C aufgeschmolzen, mit 80°C heißem Wasser vermischt und unter starkem Rühren emulgiert. Danach wurde die Emulsion unter schwachem Rühren abgekühlt.

| *Teilmischung B* | |
|---|---|
| Ammoniumsulfat | 1,00g |
| Direktziehender Farbstoff gemäß Formel (I) | 1,00g |
| Ammoniak (25%ige Lösung) | ad pH=9,0 |
| Wasser | 10,00g |

Der Farbstoff wurde in 50°C heißem Wasser unter Zugabe von Ammoniumsulfat und Ammoniak gelöst.

Die Farbstofflösung (Teilmischung B) wurde zur Emulsion (Teilmischung A) gegeben, mit Ammoniak auf pH=9 eingestellt und mit Wasser auf 100g aufgefüllt. Es wurde bis zum Erreichen der Raumtemperatur weitergerührt.

Die so erhaltene Färbecreme wurde auf 5cm lange Strähnen standardisierten, zu 80% ergrauten, aber nicht besonders vorbehandelten Menschenhaar aufgetragen, und dort 30 Minuten bei 32°C belassen. Danach wurde das Haar gespült, mit einem üblichen Haarwaschmittel ausgewaschen und anschließend getrocknet.

Das Ergebnis der Färbeversuche ist der Tabelle I zu entnehmen.

| Direktziehender Farbstoff | Nuance des gefärbten Haares |
|---|---|
| 1-(N-cycloheptylamino)-2-nitro-4-aminobenzol | Graurot |
| 1-(N-cyclooctylamino)-2-nitro-4-aminobenzol | Mattrot |

## Patentansprüche

1. Mittel zum Färben und Tönen keratinischer Fasern, insbesondere menschlichen Haaren, **dadurch gekennzeichnet, daß** es als direktziehenden Farbstoff mindestens ein 2-Nitro-p-Phenylendiaminderivat der Formel (I), 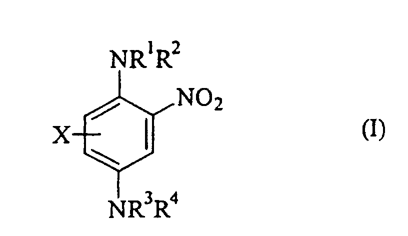 in der R¹ bis R⁴ voneinander unabhängig stehen für Wasserstoff, eine C₁₋₄-Hydroxyalkylgruppe oder einen gesättigten, einfach oder mehrfach ungesättigten C₇₋₈-Ring, der gegebenenfalls mit einer C₁₋₄-Alkylgruppe, einem Halogenatom, einer Hydroxygruppe oder einer Aminogruppe substituiert sein kann, und X steht für Wasserstoff oder ein Halogenatom, mit der Maßgabe, daß mindestens einer der Substituenten R¹ bis R⁴ ein C₇₋₈-Ring ist, enthält.

2. Mittel nach Anspruch 1, **dadurch gekennzeichnet, daß** R¹ ein Cycloheptylring ist.

3. Mittel nach Anspruch 1, **dadurch gekennzeichnet, daß** R¹ ein Cyclooctylring ist.

4. Mittel nach einem der Ansprüche 1 bis 3, **dadurch gekennzeichnet, daß** X für Wasserstoff steht.

5. Mittel nach einem der Ansprüche 1 bis 4, **dadurch gekennzeichnet, daß** R² bis R⁴ für Wasserstoff stehen.

6. Mittel nach einem der Ansprüche 1 bis 5, **dadurch gekennzeichnet, daß** es als Verbindung der Formel (I) 1-(N-cycloheptylamino)-2-nitro-4-aminobenzol enthält.

7. Mittel nach einem der Ansprüche 1 bis 6, **dadurch gekennzeichnet, daß** es als Verbindung der Formel (I) 1-(N-cyclooctylamino)-2-nitro-4-aminobenzol enthält.

8. Mittel nach einem der Ansprüche 1 bis 7, **dadurch gekennzeichnet, daß** es frei von Oxidationsfarbstoffvorprodukten ist.

9. Mittel nach Anspruch 8, **dadurch gekennzeichnet, daß** es auf dem Haar verbleibt.

10. Mittel nach einem der Ansprüche 8 oder 9, **dadurch gekennzeichnet, daß** es außerdem eine haarfestigende Funktion ausübt.

11. Mittel nach einem der Ansprüche 1 bis 7, **dadurch gekennzeichnet, daß** es mindestens eine Entwicklerkomponente enthält.

12. Mittel nach Anspruch 11, **dadurch gekennzeichnet, daß** die Entwicklerkomponente ausgewählt ist aus p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

13. Mittel nach einem der Ansprüche 11 oder 12, **dadurch gekennzeichnet, daß** es mindestens eine Kupplerkomponente, ausgewählt aus der Gruppe, die von 1-Naphthol, 1,5-, 2,7- und 1,7-Dihydroxynaphthalin, 3-Aminophenol, 5-Amino-2-methylphenol, Resorcin, 4-Chlonresorcin, 2-Chlor-6-methyl-3-aminophenol, 2-Methylresorcin, 5-Methylresorcin, 2,5-Dimethylresorcin und 2,6-Dihydroxy-3,4-diaminopyridin gebildet wird, enthält.

14. Mittel nach einem der Ansprüche 1 bis 13, **dadurch gekennzeichnet, daß** es weiterhin ein anionisches, nichtionogenes oder kationisches Polymeres enthält.

15. Verwendung eines Mittels nach einem der Ansprüche 1 bis 14 zum Färben und Tönen von Haaren.

16. 1-(N-cycloheptylamino)-2-nitro-4-aminobenzol.

17. 1-(N-cyclooctylamino)-2-nitro-4-aminobenzol.

## Claims

1. A preparation for colouring keratin fibres, more particularly human hair, **characterized in that** it contains as a substantive dye at least one 2-nitro-p-phenylenediamine derivative corresponding to formula (I): 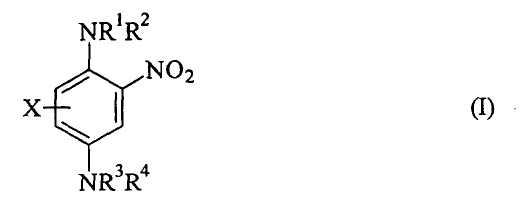 in which R¹ to R⁴ independently of one another represent hydrogen, a C₁₋₄ hydroxyalkyl group or a saturated, mono- or polyunsaturated C₇₋₈ ring which may optionally be substituted by a C₁₋₄ alkyl group, a halogen atom, a hydroxy group or an amino group and X is hydrogen or a halogen atom, with the proviso that at least one of the substituents R¹ to R⁴ is a C₇₋₈ ring.

2. A preparation as claimed in claim 1, **characterized in that** R¹ is a cycloheptyl ring.

3. A preparation as claimed in claim 1, **characterized in that** R¹ is a cyclooctyl ring.

4. A preparation as claimed in any of claims 1 to 3, **characterized in that** X stands for hydrogen.

5. A preparation as claimed in any of claims 1 to 4, **characterized in that** R² to R⁴ stand for hydrogen.

6. A preparation as claimed in any of claims 1 to 5, **characterized in that** it contains 1-(N-cycloheptylamino)-2-nitro-4-aminobenzene as the compound corresponding to formula (I).

7. A preparation as claimed in any of claims 1 to 6, **characterized in that** it contains 1-(N-cyclooctylamino)-2-nitro-4-aminobenzene as the compound corresponding to formula (I).

8. A preparation as claimed in any of claims 1 to 7, **characterized in that** it is free from oxidation dye precursors.

9. A preparation as claimed in claim 8, **characterized in that** it remains on the hair.

10. A preparation as claimed in claim 8 or 9, **characterized in that** it also performs a hair-setting function.

11. A preparation as claimed in any of claims 1 to 7, **characterized in that** it contains at least one primary intermediate.

12. A preparation as claimed in claim 11, **characterized in that** the primary intermediate is selected from p-phenylenediamine, p-toluylenediamine, p-aminophenol, 1-(2'-hydroxyethyl)-2,5-diaminobenzene, N,N-bis-(2-hydroxyethyl)-p-phenylenediamine, 4-amino-3-methylphenol, 4-amino-2-((diethylamino)-methyl)-phenol, 2-aminomethyl-4-aminophenol, 2,4,5,6-tetraaminopyrimidine, 2-hydroxy-4,5,6-triaminopyrimidine, 4-hydroxy-2,5,6-triaminopyrimidine and 4,5-diamino-1-(2'-hydroxyethyl)-pyrazole.

13. A preparation as claimed in claim 11 or 12, **characterized in that** it contains at least one secondary intermediate selected from the group consisting of 1-naphthol, 1,5-, 2,7- and 1,7-dihydroxynaphthalene, 3-aminophenol, 5-amino-2-methylphenol, resorcinol, 4-chlororesorcinol, 2-chloro-6-methyl-3-aminophenol, 2-methyl resorcinol, 5-methyl resorcinol, 2,5-dimethyl resorcinol and 2,6-dihydroxy-3,4-diaminopyridine.

14. A preparation as claimed in any of claims 1 to 13, **characterized in that** it additionally contains an anionic, nonionic or cationic polymer.

15. The use of the preparation claimed in any of claims 1 to 14 for coloring and tinting hair.

16. 1-(N-cycloheptylamino)-2-nitro-4-aminobenzene.

17. 1-(N-cyclooctylamino)-2-nitro-4-aminobenzene.

## Revendications

1. Agent de coloration et de teinture de fibres kératiniques, en particulier de cheveux humains, **caractérisé en ce qu'**il contient, à titre de colorant montant directement sur la fibre, au moins un dérivé de la 2-nitro-p-phénylènediamine répondant à la formule (I) 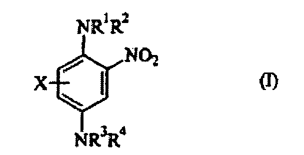 dans laquelle R¹ à R⁴ représentent, indépendamment l'un de l'autre, un atome d'hydrogène, un groupe hydroxyalkyle en C₁-C₄, ou encore un noyau en C₇-C₈ saturé, une fois insaturé ou plusieurs fois insaturé, qui peut être substitué, le cas échéant, avec un groupe alkyle en C₁-C₄, avec un atome d'halogène, avec un groupe hydroxyle ou avec un groupe amino, et X représente un atome d'hydrogène ou un atome d'halogène, avec cette mesure qu'au moins un des substituants R¹ à R⁴ représente un noyau en C₇-C₈.

2. Agent selon la revendication 1, **caractérisé en ce que** R¹ représente un noyau cycloheptyle.

3. Agent selon la revendication 1, **caractérisé en ce que** R¹ représente un noyau cyclooctyle.

4. Agent selon l'une quelconque des revendications 1 à 3, **caractérisé en ce que** X représente un atome d'hydrogène.

5. Agent selon l'une quelconque des revendications 1 à 4, **caractérisé en ce que** R² à R⁴ représentent un atome d'hydrogène.

6. Agent selon l'une quelconque des revendications 1 à 5, **caractérisé en ce qu'**il contient, à titre de composé répondant à la formule (I), le 1-(N-cycloheptylamino)-2-nitro-4-aminobenzène.

7. Agent selon l'une quelconque des revendications 1 à 6, **caractérisé en ce qu'**il contient, à titre de composé répondant à la formule (I), le 1-(N-cyclooctylamino)-2-nitro-4-aminobenzène.

8. Agent selon l'une quelconque des revendications 1 à 7, **caractérisé en ce qu'**il est exempt de précurseurs de colorant d'oxydation.

9. Agent selon la revendication 8, **caractérisé en ce qu'**il reste sur les cheveux.

10. Agent selon l'une quelconque des revendications 8 ou 9, **caractérisé en ce qu'**il exerce en outre une fonction de fixateur pour les cheveux.

11. Agent selon l'une quelconque des revendications 1 à 7, **caractérisé en ce qu'**il contient au moins un composant développeur.

12. Agent selon la revendication 11, **caractérisé en ce que** le composant développeur est choisi parmi le groupe comprenant la p-phénylènediamine, la p-toluylènediamine, le p-aminophénol, le 1-(2'-hydroxyéthyl)-2,5-diaminobenzène, la N,N-bis-(2-hydroxyéthyl)-p-phénylènediamine, le 4-amino-3-méthylphénol, le 4-amino-2-((diéthylamino)-méthyl)-phénol, le 2-aminométhyl-4-aminophénol, la 2,4,5,6-tétraaminopyrimidine, la 2-hydroxy-4,5,6-triamino-pyrimidine, la 4-hydroxy-2,5,6-triaminopyrimidine et le 4,5-diamino-1-(2'-hydroxyéthyl)-pyrazole.

13. Agent selon une quelconque des revendications 11 ou 12, **caractérisé en ce qu'**il contient au moins un composant de couplage, choisi parmi le groupe qui est formé par le 1-naphtol, le 1,5-, le 2,7- et le 1,7-dihydroxynaphtalène, le 3-aminophénol, le 5-amino-2-méthylphénol, le résorcinol, le 4-chlororésorcinol, le 2-chloro-6-méthyl-3-amino-phénol, le 2-méthylrésorcinol, le 5-méthylrésorcinol, le 2,5-diméthyl-résorcinol et la 2,6-dihydroxy-3,4-diaminopyridine.

14. Agent selon l'une quelconque des revendications 1 à 13, **caractérisé en ce qu'**il contient en outre un polymère anionique, non ionogène ou cationique.

15. Utilisation d'un agent selon l'une quelconque des revendications 1 à 14, pour la coloration et la teinture de cheveux.

16. 1-(N-cycloheptylamino)-2-nitro-4-aminobenzène.

17. 1-(N-cyclooctylamino)-2-nitro-4-aminobenzène.
